# EUROPÄISCHE PATENTSCHRIFT

(11) **EP 1 103 808 B1**
(45) Veröffentlichungstag und Bekanntmachung des Hinweises auf die Patenterteilung: **09.06.2010**
(21) Anmeldenummer: 00124843.4
(22) Anmeldetag: 14.11.2000
(51) Int. Cl.: G01N 27/414

(54) **Gassensor**
Gas sensor
Capteur de gaz

(30) Priorität: 25.11.1999 DE 19956806
(43) Veröffentlichungstag der Anmeldung: 30.05.2001
(73) Patentinhaber: Micronas GmbH, 79108 Freiburg i. Br. (DE)
(72) Erfinder: Fleischer, Maximilian, Dr., 85635 Höhenkirchen (DE); Meixner, Hans, Prof., 85540 Haar (DE); Ostrick, Bernhard, 81541 München (DE)
(74) Vertreter: Göhring, Robert

(56) Entgegenhaltungen:
- EP-A- 0 740 151
- EP-A- 0 947 829
- WO-A-99/49507
- WO-A-99/51975
- DE-A- 4 239 319
- DE-A- 19 814 857
- US-A- 4 730 479

## Beschreibung

Die Erfindung betrifft einen Gassensor gemäß dem Oberbegriff des Anspruchs 1, bei dem unter Ausnutzung der Eigenschaften eines Feldeffekttransistors die Austrittsarbeit an einer gassensitiven Schicht gemessen werden kann. Feldeffekttransistoren bestehen aus einem Source-, einem Drain- und einem Kanalbereich, wobei die gassensitive Schicht in eine Gate-Elektrode integriert sein kann und die Änderung der Austrittsarbeit des Gates des Transistors eine Änderung der Einsatzspannung des Transistors hervorruft.

Seit kurzem sind Gassensoren bekannt, die zur Detektion von Gasen die Messung der Austrittsarbeit an der Oberfläche einer gassensitiven Schicht als Sensorsignal verwenden. Diese Gassensoren werden bei Betriebstemperaturen zwischen ca. 25 bis max. 120 °C betrieben. Ihr Betrieb ist somit mit einem relativ niedrigen Energieverbrauch von meist weniger als 100 mW verbunden. Sie sind durch ein großes Spektrum nachweisbarer Gase gekennzeichnet. Durch die Verwendung der Meßgröße Austrittsarbeit ist neben der Senkung des Energieverbrauches auch eine deutliche Vereinfachung der Materialpräparation gegeben. Da nun durch die Auswertung einer reinen Oberflächeneigenschaft keine im allgemeinen schwierig zu reproduzierenden Abhängigkeit von Korngrenzen oder Elektrodenkontakten vorliegen, wie beispielsweise bei der Messung der Leitfähigkeit, sind deutlich verbesserte Reproduzierbarkeiten der Sensorsignale möglich.

Die Realisierung eines Gas-Feldeffekttransistors (GasFET), bei dem zwischen Gate-Elektrode und Kanalbereich des Transistors ein kleiner Luftspalt (0,5 - 5 um) vorhanden ist, sieht vor, daß die dem Luftspalt zugewandte Seite der Gate-Elektrode mit dem sensitiven Material beschichtet ist. Eine Änderung der Austrittsarbeit an diese Oberfläche koppelt über diesen kleinen Luftspalt kapazitiv in den Kanalbereich ein und bewirkt eine Änderung der Einsatzspannung des Transistors. Diese Änderung der Einsatzspannung ist letztlich die gemessene elektrische Größe.

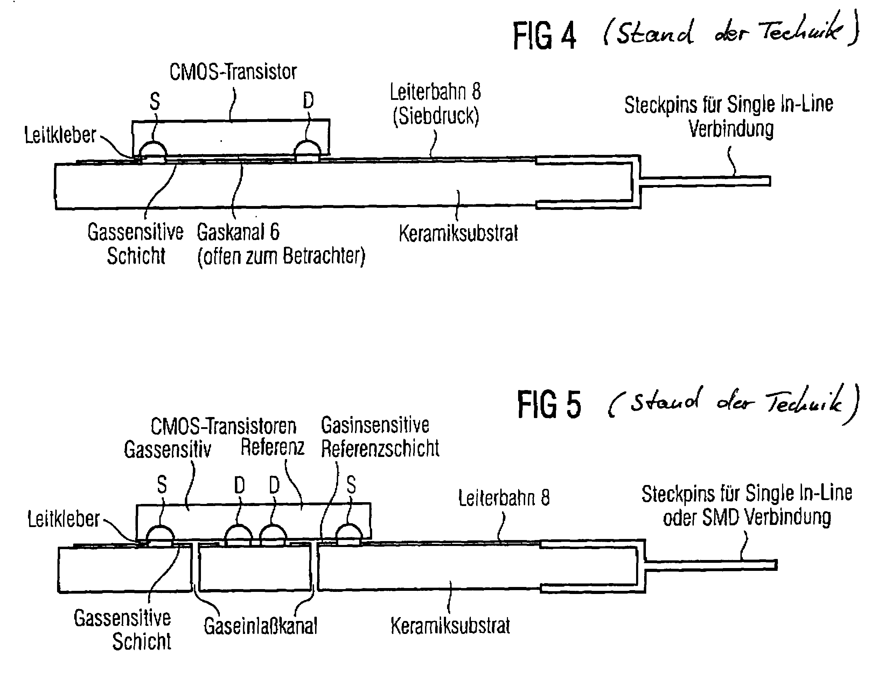

Es ist notwendig, einen Aufbau zu finden, der in geeigneter Form die Änderung der Austrittsarbeit eines Detektionsmateriales in den Kanal eines Transistors einkoppelt. Die weitaus meisten Anwendungen zielen auf ein einziges Ziel dorthin. Störend wirken sich jedoch oft
Querempfindlichkeiten der sensitiven Schicht aus, die zu eliminieren sind. Eine häufige Querempfindlichkeit ist die Sensitivität auf Luftfeuchtigkeit. So ist beispielsweise bei der Ozondetektion mit Halogenidsalzen eine starke feuchte Querempfindlichkeit zu verzeichnen, die durch das Verwenden einer zweiten, nur Feuchtigkeit detektierenden Schicht eliminiert werden kann. Dabei wird in einem benachbarten Feldeffekttransistor eine Referenzschicht zur Eliminierung der Feuchtigkeit eingesetzt. Andere Anwendungsfälle erfordern die Detektion mehrerer Gase gleichzeitig. Zur Realisierung kostengünstiger Bauelemente sind hybride Aufbauten angewandt worden, bei denen auf ein Substrat eine gassensitive Schicht aufgebracht wird, parallel dazu ein Feldeffekttransistor prozessiert wird, und diese beiden Teile dann in geeigneter Weise verbunden werden, so daß die gassensitive Schicht durch einen Luftspalt getrennt über den Kanal des Feldeffekttransistors angebracht ist. Dieses Grundprinzip wird in der deutschen Patentschrift DE 42 39 319 C2 beschrieben, wobei ein hierfür geeigneter mikromechanischer Aufbau angegeben wird. Das Grundkonzept eines industriell fertigbaren hybriden Flip-Chip-Aufbaus (unter Verwendung von Keramik- und Siliziumbauteilen) wird in der deutschen Patentanmeldung mit der amtlichen Anmeldenummer P 198 14 857.7 beschrieben. Dieser Stand der Technik wird in den Figuren 4 und 5 wiedergegeben. Gezeigt wird dabei der Querschnitt eines Designs für einen gas-sensitiven FET in Flip-Chip-Technik. Im Vordergrund steht hierbei die Suche nach einer kostengünstigen Verbindungstechnik. Als Verbindungstechnik wäre beispielsweise die Flip-Chip-Montage einsetzbar. Dabei wird ein Halbleiterbauelement, insbesondere ein CMOS-Transistor mit seiner Frontseite auf ein eine Gate-Elektrode mit einer gassensitiven Schicht beinhaltendes Keramiksubstrat aufgebracht. Dabei sind entsprechende Zuleitungen und Kontaktierungselemente für die innere Struktur und zur Außenkontaktierung vorgesehen. Somit wird prinzipiell eine industrielle Nutzung der Sensoridee erzielt. Eine technische Lösung zur Realisierung der präzisen Einstellung des benötigten Luftspaltes hinsichtlich des Abstandes zwischen FET und gassensitiver Schicht kann verschiedenartig durchgeführt werden. Sämtliche bisher bekannten Lösungen geben jedoch keinen Aufbau an, bei dem mehrere sensitive Schichten gleichzeitig ausgelesen werden können. Für das Auslesen einer sensitiven Schicht und einer gasinsensitiven Schicht wird in der genannten deutschen Patentanmeldung P 198 14 857.7 beschrieben. Dabei ist dem ein Gas detektierenden Feldeffekttransistor ein Referenztransistor benachbart zugeordnet. Auf einem einstückigen Keramiksubstrat ist nach wie vor ein einziges Halbleiterbauelement geordnet und befestigt worden. Elektrische Verbindungen sind in Form von Leiterbahnen, Anschlußflecken und Leitkleber Verbindungen eingerichtet. Der Querschnitt eines derartigen Designs mittels eines Transistors zur Kompensation von Temperatureinflüssen oder Querempfindlichkeiten ist in Figur 5 dargestellt.

Der Erfindung liegt die Aufgabe zugrunde, mindestens zwei Gase in einer Anordnung gleichzeitig detektieren zu können, wobei ein kostengünstiger Aufbau in Flip-Chip-Technologie einzusetzen ist. Desweiteren ist ein Herstellungsverfahren anzugeben.

Die Lösung dieser Aufgaben geschieht durch die Merkmalskombination entsprechend Anspruch 1. Weiterbildungen können den Unteransprüchen entnommen werden.

Das Siliziumbauelement beinhaltet beispielsweise zwei Feldeffekttransistoren, deren Kanal über den sensitiven Schichten positioniert ist. Dabei ist die Einhaltung eines vorgegebenen Abstandes zwischen Kanalbereich des Transistors und gassensitiver Schicht, beispielsweise durch Abstandshalter wesentlich. Der definierte Luftspalt zwischen Kanalbereich des Transistors und der gassensitiven Schicht muß im um-Bereich liegen und reproduzierbar sein. Dabei ist zu berücksichtigen, daß gassensitive Schichten, die beispielsweise durch Siebdrucktechnik oder Spray-Coating präpariert worden sind, typische Schichtdicken von beispielsweise 5 bis 20 um aufweisen, wobei die Reproduzierbarkeit nicht sehr hoch ist. Somit können identische Schichtdicken zwischen den beiden sensitiven Schichten schlecht dargestellt werden. In der Praxis würde dies dazu führen, daß ca. 5 um Höhendifferenz zwischen den Schichten auftreten könnten. Gassensoren mit unterschiedlicher Luftspaltbreite können jedoch an der Praxis durch das entsprechend unterschiedliche Regelverhalten der beiden Kanäle nicht als Gassensor verwendet werden.

Eine der Erfindung zu Grunde liegende Erkenntnis führt zur Verwendung von mindestens zwei Trägersubstraten. Die damit verbundenen Vorteile bestehen ebenso in der Handhabung unterschiedlicher Höhen der Flip-Chip-Bonds, was kostengünstigere Herstellungsprozesse mit sich bringt. Darüber hinaus kann mit jedem Trägersubstrat ein Feldeffekttransistor montiert werden. Dies ermöglicht wiederum die planparallele Montage, da bei jeder Vervollständigung eines Feldeffekttransistors durch die Flip-Chip-Montage das Gate mit der entsprechenden gassensitiven oder gasinsensitiven Schicht mit seinem entsprechenden Trägersubstrat separat montierbar ist. Bei mehreren Trägersubstraten besteht die Möglichkeit, die einzelnen Sensorschichten in unabhängigen und sich nicht gegenseitig beeinflußenden Herstellungsprozessen zu erzeugen.

Zur Herstellung eines Gassensors (A) kann während des Flip-Chip-Bond-Vorganges das Halbleiterbauelement soweit aufgepreßt werden, bis es auf den beiden höchsten sensitiven Schichten aufliegt.

Zur Herstellung eines Gassensors (B) wird jeweils einzeln ein Trägersubstrat mit einem im Halbleiterbauelement integrierten Feldeffekttransistor kombiniert bzw. montiert. In der Regel werden die verschiedenen Trägersubstrate unterschiedlich groß sein. Insbesondere wird ein großes Trägersubstrat vorhanden sein, welches verschiedene Flip-Chip-Bonds zur Kontaktierung während des Montagevorganges und verschiedene andere Leiterstrukturen aufweist. Auf diesem einen Trägersubstrat wird ein Feldeffekttransistor plaziert. Weitere Feldeffekttransistoren, die im Halbleiterbauelement integriert sind, korrespondieren mit weiteren separaten Trägersubstraten. Diese weiteren Trägersubstrate können kleiner sein als das erstgenannte, da sie mit geringeren Kontaktiermitteln auskommen. Auch hierbei ist jeweils ein Trägersubstrat verbunden mit einem einzigen Feldeffekttransistor. Diese kleineren Trägersubstrate können in größerer Anzahl mit einem einzigen großen Trägersubstrat kombiniert werden. Allen Varianten ist gemeinsam, daß ein übergreifendes Halbleiterbauelement vorliegt.

Im folgenden werden anhand von die Erfindung nicht einschränkenden schematischen Figuren Ausführungsbeispiele beschrieben.
- Figur 1: zeigt ein Halbleiterbauelement aus Silizium, wobei ein einziges Trägersubstrat vorhanden ist und mehrere Feldeffekttransistoren mit jeweils sensitiver Schicht dargestellt sind.
- Figur 2: zeigt eine Anordnung entsprechend Figur 1 mit zwei Trägersubstraten.
- Figur 3: zeigt eine Anordnung entsprechend Figur 2 mit einer deutlicheren Darstellung der Feldeffekttransistoren.
- Figur 4: zeigt den Stand der Technik, wobei der Querschnitt eines Designs eines gassensitiven Feldeffekttransistors dargestellt ist.
- Figur 5: zeigt den Stand der Technik, wobei ein einziges Keramiksubstrat vorliegt, in das Gaseinlaß-Kanäle eingearbeitet sind, sowie einen Referenztransistor.
- Figur 6: zeigt in der Draufsicht eine Variante mit zwei Trägersubstraten unterschiedlicher Größe.
- Figur 7: zeigt in der Draufsicht zwei Varianten a und b mit jeweils zwei Trägersubstraten,
- Figur 8: zeigt eine Anordnung mit drei Trägersubstraten.

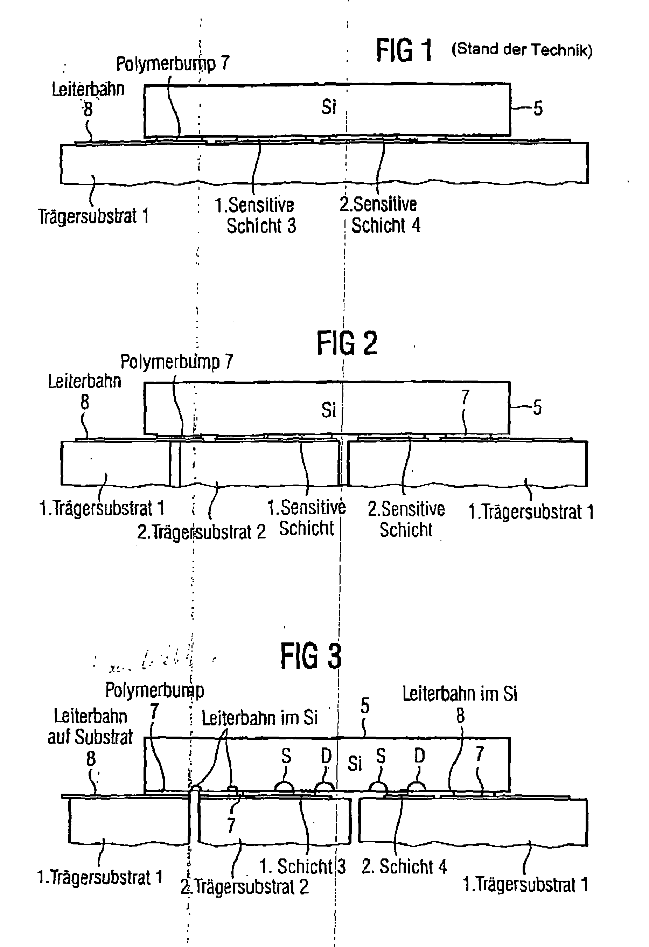

In der Figur 1 wird ein gemeinsames einziges Trägersubstrat dargestellt, das beispielsweise aus Keramik (Aluminiumoxid oder Aluminiumnitrid) oder aus Glas oder aus einem elektrisch nicht leitenden Polymer besteht. Diese Materialwahl gilt auch für andere Varianten. Das Siliziumbauelement beinhaltet ISFET-Bauteile (Feldeffekttransistor mit Isolatorschicht), deren Kanal über den sensitiven Schichten, die über dem Trägersubstrat aufgebracht sind, positioniert sind. Bei dieser Variante ist die Realisierung des Abstandshalters wichtig. So wird der Siliziumtransistor direkt mit dem Abstandshalter auf der Sensorschicht aufliegen. Dabei wird berücksichtigt, daß die Sensorschicht oft in Siebdrucktechnik oder mit Spray-Coating präpariert ist. Typische Schichtdicken liegen bei 5 bis 20 um, wobei die Reproduzierbarkeiten nicht sehr hoch sind. Somit sind identische Schichtdicken zwischen den beiden sensitiven Schichten schlecht darstellbar. In der Praxis würde dies dazu führen, daß ca. 5 um Höhendifferenz zwischen den Schichtdicken auftreten können. Nachdem typische Spaltabstände zwischen Gate und Halbleiterbauelement bei 3 um liegen, ergeben sich bei planparalleler Montage von Halbleiterchip und Trägersubstrat wesentlich unterschiedliche Luftspalthöhen, so daß das Regelverhalten der beiden Kanäle weit auseinander liegt. Für die in Figur 1 dargestellte Variante ist bei der Montage im Gegensatz zu herkömmlichen Flip-Chip-Verfahren ein adaptives Aufsetzen des Siliziumbauelementes auf das Trägersubstrat vorgesehen. Dies bedeutet, daß das Silizium nicht planparallel geführt wird, sondern es wird eine gewisse Verkippung zugelassen. Der Aufsetzvorgang läuft dergestalt ab, daß das Silizium aufgepreßt wird, bis es auf den beiden sensitiven Schichten aufliegt, die die höchsten festen Bereiche des Trägersubstrates darstellen. Die nebenbei aufgebrachten Polymerbumps bestehen aus viskosem Leitkleber und werden entsprechend zusammengedrückt. Es ergeben sich Winkel zwischen 0,2° und 2° zwischen Siliziumbauelement und Trägersubstrat. Es ist anzumerken, daß ein Siliziumbauelement auf seiner Oberfläche regelmäßig eine abschließende Isolatorschicht aufweist. Somit kann eine Sensorschicht ohne Kurzschluß direkt aufsitzen.

Sind deutlich unterschiedliche Schichtdicken der Sensorschichten durch deren Herstellungsverfahren gegeben, so können diese zugelassen werden. Dieser Höhenunterschied wird durch die Verwendung mehrerer Teilsubstrate eliminiert. Die Teilsubstrate werden jeweils mit innigen Kontakten der Sensorschicht zum Silizium montiert. Ein entsprechendes Schema bietet Figur 2 in der Seitenansicht. Es werden zwei Trägersubstrate dargestellt, deren Kombination als Mutter/Kind-Variante bezeichnet werden kann. Das in Figur 2 dargestellte erste Trägersubstrat 1 wäre in diesem Fall das größere Substrat und würde mit Mutter bezeichnet. Das zweite Trägersubstrat 2 wäre das entsprechend kleinere Substrat und würde als Kind bezeichnet. Die Anzahl der Kinder ist nicht festgelegt.

In den Figuren 2 und 3 werden ähnliche Aufbauten dargestellt, wobei in Figur 3 eine detailliertere Darstellung der Feldeffekttransistoren mit Source S und Drain D vermerkt sind. Die Flip-Chip-Montage des Halbleiterbauelementes 5 auf dem ersten Trägersubstrat 1 kann, unabhängig von der Flip-Chip-Montage des zweiten Trägersubstrates auf dem Halbleiterbauelement 5 vorgenommen werden. Weiterhin eingezeichnete Einzelheiten betreffen Leiterbahnen 8 auf dem Substrat, erste und zweite sensitive Schicht 3, 4 sowie Flip-Chip-Bonds, die insbesondere Polymerbumps 7 sind. Diese Polymerbumps 7 bestehen aus einem Leitkleber. Somit sind in Figur 3 wesentliche Einzelteile der Erfindung dargestellt, mit denen die elektrischen Anschlüsse wie Source, Drain, Rückelektroden der Sensorschichten oder eventuell Bulk-Kontakte des Siliziums berücksichtigt sind. Die Kontaktierungen werden zum Muttersubstrat geführt, da dort Kontaktelemente nach außen hin angebracht sind. Es ist anzumerken, daß
1) sämtliche sensitiven oder insensitiven Schichten eine Rükkelektrode benötigen. Zum rückseitigen Anschluß der Schichten auf dem Kindsubstrat wird über den elektrisch leitenden Polymerbump 7 vom Kind auf eine Leiterbahn 8 im Silizium kontaktiert und von dort über einen weiteren elektrisch leitenden Polymerbump 8 auf das Muttersubstrat. Analog werden andere Kindsubstrate angeschlossen.
2) Die Anschlüsse von Source und Drain im Silizium geschehen über Leiterbahnen 8 im Silizium und einen elektrisch leitenden Polymerbump auf das Muttersubstrat. Nicht dargestellte elektrische Anschlüsse laufen senkrecht zur Zeichenebene. Analog wird ein eventuell vorhandener Bulk-Kontakt des Siliziums angeschlossen.

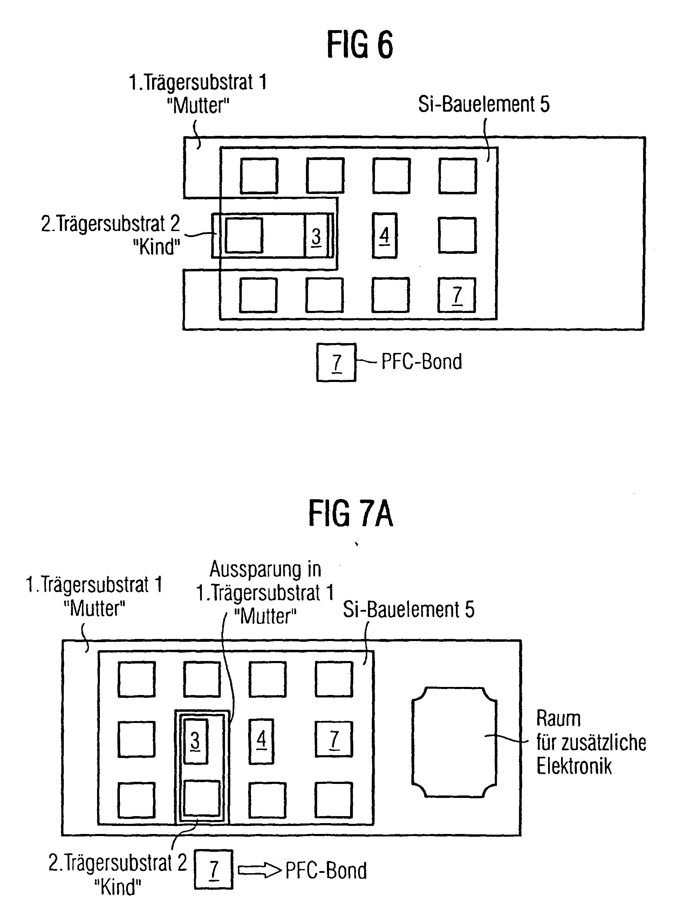

Nachdem die Figuren 2 und 3 eine erfindungsgemäße Anordnung im seitlichen Schnitt darstellen, werden in den Figuren 6, 7 und 8 Aufsichten gezeigt.

In Figur 6 ist eine Anordnung gezeigt, bei der eine Sensorschicht auf das im allgemeinen größere erste Trägersubstrat aufgebracht wird, über welches die Halterung des gesamten Bauelementes sowie in sämtlichen Fällen die Leitung elektrischer Signale nach außen hin geschieht. Auf ein zweites Trägersubstrat 2 wird die weitere Sensorschicht aufgebracht. Beide Trägersubstrate 1, 2 werden in Flip-Chip-Bond-Prozessen oder ähnlichem an das Siliziumbauelement montiert, so daß jeweils die Sensorschicht direkt am Siliziumbauelement 5 anliegt. Dadurch werden reproduzierbare Luftspalte bzw. Luftspalthöhen erzeugt.

Eine elektrische Kontaktierung der Rückelektrode von Sensorschichten des zweiten Trägersubstrates 2 an das erste Trägersubstrat 1 ist notwendig. Dies kann entweder über Drahtbrükken oder wie es in Figur 2 bzw. Figur 3 dargestellt ist, über zwei Flip-Chip-Bonds und eine entsprechende Leiterbahn im Siliziumbauelement 5 realisiert werden. Der besondere Vorteil besteht darin, daß keine Anforderungen mehr an die Gleichheit der Dicken bzw. Höhen von zwei Sensorschichten gestellt werden und daß die beiden Sensorschichten in vollkommen separaten Prozessen hergestellt werden können. Zudem ist es möglich, auf dem ersten Trägersubstrat 1 elektronische Bauteile zur Bildung einer primären Sensorelektronik unterzubringen. Dabei würde keinerlei Rücksicht bei der Montage auf etwaige Empfindlichkeiten von auf dem zweiten Trägersubstrat (Kind) angebrachten Sensorschichten genommen werden müssen. Mit anderen Worten kann etwa ein Reflow-Löten mit der damit verbundenen erheblichen Temperaturbelastung zugelassen werden.

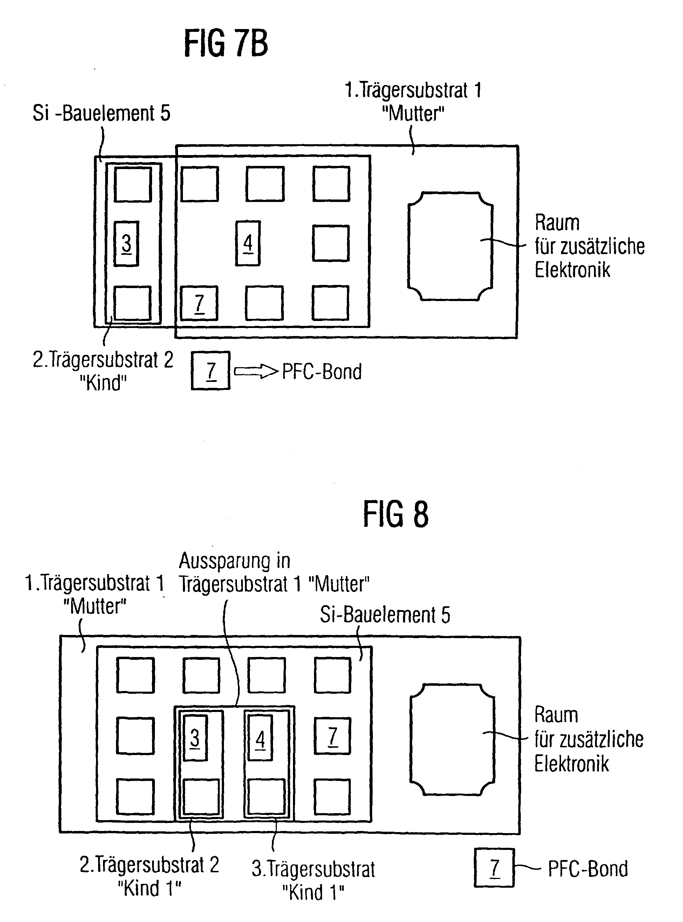

Die weitere Ausführungsform entsprechend Figur 7 zeigt in Figur 7a ein Muttersubstrat mit einer innenliegenden Aussparung und in Figur 7b eine symmetrische Befestigung des Kindsubstrates. Separat gekennzeichnet sind der Raum für zusätzliche elektronische Bauteile, sowie die Unterteilung in erstes Trägersubstrat 1 (Mutter) und zweite Trägersubstrate 2 (Kind). Das Siliziumbauelement 5 überspannt jeweils die Bereiche, in denen Montageelemente vorhanden sind oder Feldeffekttransistoren montiert werden. Unter dem PFC-Bond ist ein Polymer-Flip-Chip-Bond zu verstehen. In Figur 7b ist das Kindsubstrat, das zweite Trägersubstrat 2 mit symmetrischer Befestigung entsprechend der auf dem ersten Trägersubstrat 1 (Muttersubstrat) befindlichen Befestigungsstruktur angebracht. An diese Variante könnten im Prinzip weitere kleine Trägersubstrate (Kind) angesetzt werden, die mit gassensitiven Schichten, Referenzschichten oder auch mit Kontaktiermitteln belegt sind. In diesem Fall können eine Elektronik auf dem ersten Trägersubstrat 1 (Mutter) oder auch beide Sensorschichten unabhängig voneinander prozessiert werden. Dies ermöglicht eine größere Freiheit bei der Auswahl der Fertigungsprozesse sowie bei der Logistik.

Figur 8 zeigt eine Ausführungsform der Erfindung mit mehreren Trägersubstraten, wobei ein erstes Trägersubstrat 1 (Mutter) vorhanden ist und zwei weitere Trägersubstrate 2, 3. Die weiteren Trägersubstrate 2, 3 sind jeweils mit einer separaten sensitiven Schicht auf ein Siliziumbauteil mit der entsprechenden Zahl von Feldeffekttransistoren auf dem Siliziumchip kombiniert. Dargestellt sind wiederum Siliziumbauelement 5, das die gesamte Anordnung abdeckt, ein erstes Trägersubstrat 1, auf dem auch Raum für zusätzliche Elektronik vorhanden ist, eine Aussparung im ersten Trägersubstrat 1, in der das zweite und dritte Trägersubstrat zum liegen kommen, die Sensorschichten 3, 4 und Polymer-Flip-Chip-Bonds 7 zur Montage der Anordnung. Im Gegensatz zu den Figuren 6 und 7 ist in Figur 8 auf dem ersten Trägersubstrat 1 kein Feldeffekttransistor vorgesehen.

Die Figuren 4 und 5 stellen Stand der Technik dar, wobei jeweils ein einziges Keramiksubstrat verwendet wird. Die in Figur 5 dargestellten senkrechten Gaseinlaßkanäle bedeuten keine Teilung des Keramiksubstrates, sondern sind lediglich Durchbrüche im Keramiksubstrat, um eine Zirkulation der Gasatmosphäre zur gassensitiven Schicht zuzulassen.

## Patentansprüche

1. Gassensor (B), aufweisend:
- ein Halbleiterbauelement (5) mit mindestens zwei integrierten Feldeffekttransistoren, welche jeweils einen Source-, einen Drain- und einen Kanalbereich aufweisen;
- mindestens zwei gassensitive Schichten (3, 4) mit jeweils zugehöriger Gate-Elektrode, in die jeweils eine der gassensitiven Schichten integriert ist;
- wobei das Halbleiterbauelement (5) an wenigstens einem Trägersubstrat (1,2) derart frontseitig angelegt, positioniert und fixiert ist, dass Gate-Elektroden und FET-Kanäle zueinander ausgerichtet sind und dass jede der gassensitiven Schichten (3, 4) einem Feldeffekttransistor zugewandt und von dem zugehörigen Kanalbereich beabstandet ist; und
- Mittel, welche zum separaten Auslesen der mindestens zwei gassensitiven Schichten (3, 4) geeignet sind,
**dadurch gekennzeichnet, dass**
von den mindestens zwei gassensitiven Schichten (3, 4) wenigstens zwei zusammen mit der jeweils zugehörigen Gate-Elektrode auf separaten Trägersubstraten (1, 2) angeordnet sind.

2. Gassensor nach Anspruch 1,
**dadurch gekennzeichnet, dass** wenigstens ein Trägersubstrat (1, 2) Elektronik- und Kontaktiermittel aufweist, welche zum Anschluss an eine Elektronikeinheit geeignet sind.

3. Gassensor nach einem der vorhergehenden Ansprüche,
**dadurch gekennzeichnet, dass** das Halbleiterbauelement (5) ein Silizium-Bauelement ist.

4. Gassensor nach einem der vorhergehenden Ansprüche,
**dadurch gekennzeichnet, dass** ein Trägersubstrat (1, 2) aus Keramik besteht.

5. Gassensor nach einem der vorhergehenden Ansprüche
**dadurch gekennzeichnet, dass** das Halbleiterbauelement (5) an einem Trägersubstrat (1, 2) mittels Flip-Chip-Montage angelegt, positioniert und fixiert ist.

6. Gassensor nach Anspruch 5,
**dadurch gekennzeichnet , dass** als Flip-Chip-Bonds leitende Polymerbumps (7) vorgesehen sind.

7. Gassensor nach einem der vorhergehenden Ansprüche,
**dadurch gekennzeichnet, dass** unterschiedliche Trägersubstrate (1, 2) unterschiedliche Größen aufweisen.

## Claims

1. Gas sensor (B), having:
- a semiconductor component (5) comprising at least two integrated field effect transistors which in each case have a source region, a drain region and a channel region;
- at least two gas-sensitive layers (3, 4), each having an associated gate electrode in which in each case one of the gas-sensitive layers is integrated;
- wherein the semiconductor component (5) is applied, positioned and fixed on the front of at least one carrier substrate (1, 2) in such a way that the gate electrodes and the FET channels are aligned with one another and that each of the gas-sensitive layers (3, 4) faces towards a field effect transistor and is spaced apart from the associated channel region; and
- means which are suitable for separately reading the at least two gas-sensitive layers (3, 4),
**characterised in that**, of the at least two gas-sensitive layers (3, 4), at least two are arranged together with the respectively associated gate electrode on separate carrier substrates (1, 2).

2. Gas sensor according to claim 1, **characterised in that** at least one carrier substrate (1, 2) has electronic and contacting means which are suitable for connection to an electronic unit.

3. Gas sensor according to one of the preceding claims, **characterised in that** the semiconductor component (5) is a silicon component.

4. Gas sensor according to one of the preceding claims, **characterised in that** one carrier substrate (1, 2) is made from ceramic.

5. Gas sensor according to one of the preceding claims, **characterised in that** the semiconductor component (5) is applied, position and fixed on a carrier substrate (1, 2) by means of a flip-chip mounting technique.

6. Gas sensor according to claim 5, **characterised in that** conductive polymer bumps (7) are provided as flip-chip bonds.

7. Gas sensor according to one of the preceding claims, **characterised in that** different carrier substrates (1, 2) have different sizes.

## Revendications

1. Capteur de gaz (B) comportant :
- un composant semi-conducteur (5) ayant au moins deux transistors à effet de champ, intégrés, ayant chacun une zone de source, une zone de drain et une zone de canal,
- au moins deux couches sensibles aux gaz (3, 4) ayant chacune une électrode de porte, associée, dans laquelle est intégrée respectivement une des couches sensibles aux gaz,
- le composant semi-conducteur (5) étant appliqué contre au moins un substrat de support (1, 2) sur la face frontale de celui-ci, en étant positionné et fixé de façon que les électrodes de porte et les canaux FET, soient alignés et que chacune des couches sensibles aux gaz (3, 4), soit tournée vers un transistor à effet de champ en étant écartée de la zone de canal correspondante, et
- des moyens permettant de lire séparément au moins deux couches sensibles aux gaz (3, 4),
**caractérisé en ce que**
sur au moins les deux couches sensibles aux gaz (3, 4), au moins deux sont installées avec l'électrode de porte correspondante, respective, sur un substrat de support (1, 2), distinct.

2. Capteur de gaz selon la revendication 1,
**caractérisé en ce qu'**
au moins un substrat de support (1, 2) comporte des moyens électroniques et des moyens de contact servant au branchement d'une unité électronique.

3. Capteur de gaz selon l'une des revendications précédentes,
**caractérisé en ce que**
le composant semi-conducteur (5) est un composant au silicium.

4. Capteur de gaz selon l'une des revendications précédentes,
**caractérisé en ce qu'**
un substrat de support (1, 2) est en céramique.

5. Capteur de gaz selon l'une des revendications précédentes,
**caractérisé en ce que**
le composant semi-conducteur (5) est appliqué, positionné et fixé sur un substrat de support (1, 2) par un montage flip-chip.

6. Capteur de gaz selon la revendication 5,
**caractérisé en ce que**
des bossages en polymère conducteur (7) sont prévus comme bossages flip-chip.

7. Capteur de gaz selon l'une des revendications précédentes,
**caractérisé en ce que**
des substrats de support différents (1, 2), ont des dimensions différentes.
